# EUROPÄISCHE PATENTANMELDUNG

(11) **EP 4 834 864 A1**
(43) Veröffentlichungstag der Anmeldung: **04.11.2026**
(21) Anmeldenummer: 26171590.8
(22) Anmeldetag: 10.04.2026
(51) Int. Cl.: C04B 35/486, B32B 18/00

(54) **OPTISCH VERBESSERTE DENTALKERAMIK**

(30) Priorität: 17.04.2025 DE 102025115301
(71) Anmelder: Vita Zahnfabrik H. Rauter GmbH & Co. KG, 79713 Bad Säckingen (DE)
(72) Erfinder: Neun, Christopher, 79713 Bad Säckingen (DE); Gödiker, Michael, 79713 Bad Säckingen (DE)
(74) Vertreter: dompatent

(57) **Zusammenfassung**

Beschrieben wird eine dentale Restauration erhältlich durch ein Verfahren umfassend die Schritte:
a) Sintern eines keramischen Materials enthaltend Titandioxid (TiO₂) und/oder Eisenoxid und anschließendes
b) Abkühlen der gesinterten dentalen Restauration, vorzugsweise auf Raumtemperatur, wobei die Abkühlung teilweise oder vollständig kontrolliert erfolgt.

## Beschreibung

Die vorliegende Erfindung betrifft dentale Restaurationen, welche erhältlich sind durch Sintern eines keramischen Materials, enthaltend Titandioxid und/oder Eisenoxid und anschließendes kontrolliertes Abkühlen der gesinterten, dentalen Restauration. Weiterhin betrifft die Erfindung ein Verfahren zur Herstellung dentaler Restaurationen.

Oxidkeramische dentale Restaurationen haben aufgrund ihrer Härte, Festigkeit und Resistenzeigenschaften zunehmend Einzug in die Dentaltechnik erhalten. Insbesondere Zirkoniumdioxid-Keramiken haben aufgrund ihrer Härte und guten Bearbeitbarkeit sowie aufgrund ihrer steuerbaren Transluzenz-Eigenschaften an Bedeutung für die Herstellung dentaler Restaurationen gewonnen. Die Einfärbung der Oxidkeramiken, insbesondere der Zirkoniumdioxid-Keramiken, erfolgt üblicherweise durch Zusatz von färbenden anorganischen Oxiden, die zusammen mit dem Zirkoniumdioxid versintert werden. Um einen natürlichen Verlauf der Zahnfarbe zu erhalten, bietet es sich an die Konzentration der färbenden Oxide schichtweise zu verändern und so einzustellen, dass der Farbverlauf der natürlichen Zahnfarbe möglichst nahekommt. Problematisch ist hierbei jedoch, dass die färbenden Metalloxide einen Einfluss auf das Sinterverhalten der Keramiken haben können. Zur Optimierung des Sinterverhaltens kann beispielsweise Titandioxid zu den dentalkeramischen Materialien hinzugegeben werden. Es wurde jedoch festgestellt, dass die Zugabe von Titandioxid, insbesondere zu Zirconiumdioxid enthaltenden Dentalkeramiken, zu einer ungewollten Vergrauung der dentalen Restaurationen führen kann. Diese Vergrauungen sind häufig mit dem bloßen Auge erkennbar und führen daher zu einer Abweichung der gewünschten Zahnfarbe. Ohne an eine Theorie gebunden zu sein wird vermutet, dass es während oder nach dem Sinterungsprozess zur Ausbildung von Titansuboxiden kommt, die ursächlich für den Grauschleier auf den dentalen Restaurationen sein könnten. Weiterhin wurde beobachtet, dass durch die im Stand der Technik üblichen Sinterprozesse und die sich daran anschließende Abkühlung eine Verringerung der Farbintensität (Farbsättigung) bei Eisenoxidhaltigen Keramiken zu beobachten ist. Typischerweise werden dentale Restaurationen nach dem Sintervorgang durch natürliche Abkühlung auf Raumtemperatur abgekühlt. Diese Abkühlung erfolgt typischerweise dadurch, dass der Sinterofen ausgeschaltet wird und es zunächst im sintertemperaturnahen Bereich zu Abkühlraten von circa 100 °C/Minute kommt.

EP 4137312 A1 beschreibt ein Verfahren zur Herstellung einer dentalen Restauration, bei dem ein Oxidkeramikmaterial mindestens einer Wärmebehandlung unterworfen wird und abgekühlt wird, wobei das Abkühlen einen ersten Abkühlschritt mit der Abkühlrate T1 und einen zweiten Abkühlschritt mit der Abkühlrate T2 umfasst.

Der vorliegenden Erfindung liegt daher die Aufgabe zugrunde dentale Restaurationen zur Verfügung zu stellen, die die vorgenannten Probleme lösen und insbesondere eine verbesserte Farbsättigung sowie eine verminderte Vergrauung aufweisen.

Gelöst werden die zuvor genannten Aufgaben durch eine dentale Restauration gemäß Anspruch 1 der vorliegenden Erfindung.

Gegenstand der vorliegenden Erfindung in einer ersten Ausgestaltung ist eine dentale Restauration erhältlich durch ein Verfahren umfassend die Schritte:
a) Sintern eines keramischen Materials enthaltend Titandioxid (TiO₂) und/oder Eisenoxid und anschließendes
b) Abkühlen der gesinterten dentalen Restauration, wobei die Abkühlung teilweise oder vollständig kontrolliert erfolgt.

Es wurde überraschend gefunden, dass eine kontrollierte Abkühlung zumindest in Teilbereichen der Abkühlkurve, insbesondere im höheren Temperaturbereich von beispielsweise oberhalb von 800 °C, speziell oberhalb von 1000 °C, eine Verringerung der Vergrauung (Graufärbung) der dentalen Restauration bei Titandioxid enthaltenden keramischen Materialien erreicht werden kann. Darüber hinaus wurde festgestellt, dass auch bei Eisenoxid enthaltenden keramischen Dentalmaterialien eine kontrollierte Abkühlung zu einer Intensivierung der Farbe der dentalen Restaurationen führt.

Die kontrollierte Abkühlung der gesinterten dentalen Restaurationen zeichnet sich üblicherweise darin aus, dass die Abkühlung im Vergleich zum im Stand der Technik üblichen Verfahren verlangsamt wird.

In einer bevorzugten Ausgestaltung der vorliegenden Erfindung, kann das kontrollierte Abkühlen eine graduelle Abkühlung und/oder eine stufenweise Abkühlung umfassen. Üblicherweise wird das kontrollierte Abkühlen über einen Zeitraum von wenigstens 5 Minuten, vorzugsweise wenigstens 10 Minuten, besonders bevorzugt wenigstens 15 Minuten, beispielsweise 10 bis 200 Minuten, speziell 15 bis 100 Minuten durchgeführt.

Bei der graduellen kontrollierten Abkühlung wird die Abkühlrate deutlich verlangsamt. Dies kann beispielsweise über eine Temperatursteuerung und ein Abkühlprogramm des Sinterofens erzielt werden. Im Rahmen der vorliegenden Erfindung umfasst das kontrollierte Abkühlen einen Temperaturabschnitt in dem die Abkühlrate von 1 bis 25 °C/min, vorzugsweise von 2 bis 15 °C/min, insbesondere von 3 bis 10 °C/min oder 3 bis 7 °C/min erfolgt.

Teilweise kontrollierte Abkühlung bedeutet, dass die Abkühlung nur in einem oder mehreren Temperaturbereichen erfolgt, also nicht über den vollständigen Temperaturbereich von Sintertemperatur bis Raumtemperatur (20 °C). Die teilweise kontrollierte Abkühlung ist erfindungsgemäß bevorzugt, da bereits eine hervorragende Wirkung erreicht wird, wenn die Abkühlkontrolle (Steuerung) im Bereich von 50 bis 800°, speziell 80 bis 500°C unterhalb der Sintertemperatur durchgeführt wird. Eine weitergehende Abkühlkontrolle im kühleren Bereich ist aus ökonomischen Gründen, aufgrund des größeren Zeitaufwands im Verhältnis zum Nutzen ist einigen Ausführungsformen nicht erforderlich.

Die stufenweise Abkühlung kann dadurch erfolgen, dass während des Abkühlvorgangs eine bestimmte Temperatur über einen bestimmten Zeitraum gehalten wird (Isotherme). In einer bevorzugten Ausgestaltung der vorliegenden Erfindung erfolgt das kontrollierte Abkühlen stufenweise mit wenigstens einer Isotherme, insbesondere über einen Zeitraum von mehr als 5 Minuten, insbesondere von mehr als 10 Minuten oder in einem Bereich von 10 bis 45 Minuten.

Es hat sich als vorteilhaft herausgestellt, dass kontrollierte Abkühlen in einem Temperaturbereich durchzuführen, der zwischen der Sintertemperatur bis etwa 800 °C, speziell im Temperaturbereich von der Sintertemperatur bis etwa 1000 °C erfolgt.

Insbesondere in dem Hochtemperaturbereich, der nahe der Sintertemperatur liegt, ist zumindest die teilweise kontrollierte Abkühlung besonders wirksam. In einer weiteren Ausgestaltung der vorliegenden Erfindung ist das Abkühlen eine kontrollierte Reduzierung der Abkühlrate, vorzugsweise im Temperaturbereich von Sintertemperatur bis etwa 800 °C, speziell bis etwa 1000 °C.

Die für das Sintern keramischer dentaler Restaurationen üblichen Temperaturen liegen oberhalb von 1350 °C, vorzugsweise in einem Bereich von 1400 °C bis 1700 °C und insbesondere von 1420 °C bis 1650 °C.

In einer bevorzugten Ausgestaltung erfolgt das kontrollierte Abkühlen ganz oder teilweise in einem Temperaturbereich von 1400 °C bis 800 °C, besonders bevorzugt von 1350 °C bis 1000 °C.

In einer weiteren bevorzugten Ausgestaltung der vorliegenden Erfindung erfolgt das kontrollierte Abkühlen stufenweise und umfasst 2 oder mehr Isothermen mit jeweils einer Haltedauer oder einer Gesamthaltedauer von vorzugsweise mindestens 5 Minuten, besonders bevorzugt mindestens 10 Minuten und speziell bevorzugt zwischen 10 Minuten und 120 Minuten, beispielsweise 15 bis 45 Minuten.

In einer bevorzugten Ausgestaltung der vorliegenden Erfindung weist die kontrollierte Abkühlung 2 oder mehr Isothermen auf, die im Temperaturbereich zwischen 1400°C und 1000°C mit einer bevorzugten Haltedauer von 2 bis 45 Minuten, insbesondere von 5 bis 30 Minuten, durchgeführt werden.

Die dentalen Restaurationen der vorliegenden Erfindung sind erhältlich durch Sintern eines keramischen Materials, welches Titandioxid und/oder Eisenoxid enthält sowie ein anschließendes zumindest teilweise kontrolliertes Abkühlen. Das keramische Material umfasst bevorzugt oxidkeramisches Material, welches Titandioxid und/oder Eisenoxid enthält.

Als besonders geeignetes keramisches Material haben sich Zirkoniumdioxidenthaltende keramische Materialien herausgestellt.

In einer Ausgestaltung der vorliegenden Erfindung umfasst das keramische Material mindestens 85 Gew.-% ZrO₂ und/oder HfO₂, wobei die Gewichtsangabe bezogen ist auf das Gesamtgewicht des keramischen Materials.

Das keramische Material weist in einer Ausführungsform Eisenoxid, vorzugsweise Eisenoxid und Titanoxid, auf. Eisenoxide sind dem Fachmann auf dem technischen Gebiet der Dentaltechnik als anorganische Färbemittel für keramische Dentalrestaurationen bekannt. In einer bevorzugten Ausführungsform der vorliegenden Erfindung weist das keramische Material Eisenoxid in einer Menge von 0,001 bis 0,2 Gew.-%, vorzugsweise von 0,01 bis 0,18 Gew.-%, auf, wobei die Gewichtsangaben jeweils bezogen sind auf das Gesamtgewicht des keramischen Materials.

In einer weiteren Ausführungsform der vorliegenden Erfindung enthält das keramische Material Titandioxid. Es wurde gefunden, dass Titandioxid zur Verbesserung der Sintereigenschaften beiträgt, insbesondere in Gegenwart von Eisenoxid. Vorzugsweise enthält das keramische Material Titandioxid in einer Menge von 0,001 bis 1,0 Gew.-%, vorzugsweise von 0.002 bis 0.8 Gew.-%, oder 0,002 bis 0,6 Gew.-%, insbesondere von 0,005 bis 0,5 Gew.-%, wobei die Gewichtsangaben (Gew.-%) jeweils bezogen sind auf das Gesamtgewicht des keramischen Materials.

In einer bevorzugten Ausgestaltung weist das keramische Material Yttriumoxid auf. Üblicherweise liegt das Yttriumoxid in einer Menge von 6 bis 10 Gew.-%, besonders bevorzugt von 6,5 bis 9 Gew.-%, bezogen auf das Gesamtgewicht der dentalen Restauration vor.

In einer bevorzugten Ausführungsform weisen die erfindungsgemäßen dentalen Restaurationen Al₂O₃ auf, vorzugsweise mindestens in einer Menge von 0,02 Gew.-%, bezogen auf das Gesamtgewicht der Restauration.

Es hat sich als vorteilhaft herausgestellt, dass die Restauration mindestens 0,02 Gew.-% Al₂O₃, vorzugsweise Al₂O₃ in einer Menge von 0,03 bis 0,15 Gew.-%, insbesondere Al₂O₃ in einer Menge von 0,03 bis 0,08 Gew.-% aufweist. Das Al₂O₃ führt zu einem verbesserten Stabilitäts- und Festigkeitsverhalten der erfindungsgemäßen dentalen Restauration.

In einer weiteren Ausgestaltung weist das keramische Material zusätzlich Cobaltoxid (Co₃O₄) auf. Das Cobaltoxid kann als zusätzlich färbendes Pigment eingesetzt werden. Üblicherweise weist das keramische Material 0,001 bis 0,02 Gew.-% Cobaltoxid auf, wobei die Gewichtsangabe bezogen ist auf das keramische Material.

In einer weiteren Ausführungsform der vorliegenden Erfindung weist das keramische Material zusätzlich Erbiumoxid auf. Erbiumoxid kann ebenfalls zur Einstellung von keramischen Zahnfarben, insbesondere in Zirkoniumdioxidbasierten Dentalmaterialien, eingesetzt werden. In einer Ausführungsform weist das keramische Material Er₂O₃, vorzugsweise in einer Menge von 0 bis weniger 1 Gew.-%, insbesondere von 0,1 bis weniger als 0,9 Gew.-% oder von 0,2 Gew.-% bis 0,8, insbesondere von 0,25 bis 0,7 Gew.-%, speziell von 0,3 bis 0,6 Gew.-%, jeweils bezogen auf das Gesamtgewicht des keramischen Materials, auf.

Vorzugsweise weist das keramische Material zusätzlich Terbiumoxid auf. Der Einsatz von Terbiumoxid hat sich als hervorragend für die Einfärbung als auch für die Verzugsstabilität herausgestellt.

Terbiumoxid im Sinne der vorliegenden Erfindung umfasst grundsätzlich alle Oxide des Terbiums. Bevorzugt ist das Terbiumoxid ausgewählt aus der Gruppe bestehend aus Tb₂O₃, Tb₄O₇, Tb₇O₁₂, Tb₁₁O₂₀, TbO₂ und Mischung hiervon.

Besonders bevorzugt eingesetzt werden kann Terbiumoxid ausgewählt aus der Gruppe bestehend aus Tb₄O₇, Tb₇O₁₂, Tb₁₁O₂₀ und beliebige Mischungen hiervon.

Speziell bevorzugt liegt das Terbiumoxid als Tb₄O₇ oder als Mischung von Tb₄O₇ mit Tb₇O₁₂, und/oder Tb₁₁O₂₀ vor.

Bevorzugt sind zudem Terbiumoxide, die mit gemischter Valenz vorliegen.

Terbiumoxid verbessert insbesondere im Zusammenspiel mit dem Titandioxid die Sintergeschwindigkeit sowie die Sintereigenschaft. Zusätzlich trägt es zur Einfärbung des keramischen Materials bei. In einer Ausgestaltung der Erfindung weist das keramische Material Terbiumoxid in einer Menge von 0,001 bis 0,25 Gew.-%, vorzugsweise von 0,002 bis 0,19 Gew.-%, insbesondere von 0,003 bis 0,17 Gew.-%, speziell 0,01 bis 0,15 Gew.-% auf, wobei die Gewichtsangaben (Gew.-%) jeweils bezogen sind auf das Gesamtgewicht des keramischen Materials.

In einer weiteren bevorzugten Ausführungsform der vorliegenden Erfindung weist die Restauration einen Farbverlauf und/oder Transluzenzverlauf auf. Dies ist insbesondere vorteilhaft um den natürlichen Farbverlauf von Zähnen nachzubilden. Es hat sich gezeigt, dass Farb-und Transluzenzverläufe durch einen schichtweisen Aufbau des keramischen Materials erreicht werden können. Hierzu können Pressformkörper umfassend 2 oder mehr farblich unterschiedliche keramische Pulverschichten gesintert werden wodurch sich bei geeigneten anorganischen Farbpigmenten ein Farbverlauf und durch Variation des Yttriumoxidgehalts in den Schichten ein Transluzenzverlauf einstellt.

In einer bevorzugten Ausgestaltung der vorliegenden Erfindung umfasst das Sintern das Sintern eines Pressformkörpers umfassend 2 oder mehr farblich unterschiedliche keramische Pulverschichten, wobei jede Pulverschicht vorzugsweise mindestens 80 Gew.-% ZrO₂ aufweist. In bevorzugten Ausgestaltungen weist der Pressformkörper wenigstens 2 Pulverschichten oder wenigstens 3 Pulverschichten oder wenigstens 4 Pulverschichten, speziell 5 Pulverschichten auf.

In einer bevorzugten Ausführungsform umfasst wenigstens eine Pulverschicht, bevorzugt wenigstens 2 Pulverschichten oder wenigstens 3 Pulverschichten oder wenigstens 4 Pulverschichten, speziell 5 Pulverschichten oder jede Pulverschicht Terbiumoxid in einer Menge von 0,001 bis 0,15 Gew.-%, vorzugsweise 0,01 bis 0,1 Gew.-%, insbesondere 0,02 bis 0,08 Gew.-%, speziell 0,02 bis 0,06 Gew.-% Terbiumoxid.

Es hat sich überraschend gezeigt, dass jede der gesinterten Pulverschichten eine im Wesentlichen gleiche Volumenveränderung über einen Temperaturbereich von 25 bis 1600 °C, insbesondere über einen Temperaturbereich von 50 bis 1400 °C oder 900 bis 1400 °C und speziell über einen Temperaturbereich von 900 bis 1350 °C, erfährt.

Im Sinne der vorliegenden Erfindung bedeutet eine im Wesentlichen gleiche Volumenveränderung, dass die Differenz der Volumenveränderung bei einer vorgegebenen Temperatur im Bereich von 25 bis 1600 °C von 2 Schichten des erfindungsgemäßen gesinterten Rohlings maximal 1%, vorzugsweise maximal 0.5 %, insbesondere maximal 0.05 %, beträgt.

Dies führt für die erfindungsgemäßen dentalen Restaurationen zu einem geringen Sinterverzug.

In einer weiteren bevorzugten Ausgestaltung der vorliegenden Erfindung weist jede Pulverschicht eine unterschiedliche Konzentration Terbiumoxid, Erbiumoxid und Cobaltoxid auf.

In einer bevorzugten Ausgestaltung weist jede Pulverschicht Eisenoxid, Erbiumoxid und Cobaltoxid als färbende Metalloxide auf.

In einer weiteren bevorzugten Ausgestaltung der vorliegenden Erfindung ist wenigstens eine Pulverschicht, bevorzugt 2 oder mehr Pulverschichten, insbesondere alle Pulverschichten im Wesentlichen frei von Fluoreszenzeffekt erzeugenden Elementen, insbesondere im Wesentlichen frei von Bismut.

Im Wesentlichen frei von Fluoreszenzeffekt erzeugenden Elementen im Sinne der vorliegenden Erfindung bedeutet, dass weniger als 0,01 Gew.-%, vorzugsweise weniger als 0.001 Gew.-% oder weniger als 0,0001 Gew.-%, jeweils bezogen auf das Gesamtgewicht der Pulverschicht, vorliegt. In einer bevorzugten Ausgestaltung ist der gesinterte Formkörper frei von weiteren Fluoreszenzeffekt erzeugenden Metalloxiden.

Der Einsatz von Lanthanoxid in dentalen Restaurationen kann sich unvorteilhaft auf die Farbkomposition, das heißt das Zusammenspiel der einzusetzenden färbenden Metalloxide in den erfindungsgemäß einzusetzenden Pulverzusammensetzungen auswirken.

In einer weiteren bevorzugten Ausgestaltung der vorliegenden Erfindung ist daher wenigstens eine Pulverschicht, bevorzugt 2 oder mehr Pulverschichten, insbesondere alle Pulverschichten im Wesentlichen frei von Lanthanoxid.

Im Wesentlichen frei von Lanthanoxid im Sinne der vorliegenden Erfindung bedeutet, dass weniger als 0,004 Gew.-%, vorzugsweise weniger als 0.001 Gew.-% oder weniger als 0,0001 Gew.-%, jeweils bezogen auf das Gesamtgewicht der Pulverschicht, vorliegt. In einer bevorzugten Ausgestaltung ist die Restauration frei von Lanthanoxid.

Bevorzugt weist wenigstens eine Pulverschicht, besonders bevorzugt 2 oder mehr Pulverschichten, insbesondere jede Pulverschicht Erbiumoxid (Er₂O₃) in einer Menge von 0,1 bis 1,0 Gew.-%, speziell 0,2 bis 0,8 Gew.-%, jeweils bezogen auf das Gesamtgewicht der Pulverschichtzusammensetzung, auf. Es hat sich überraschend gezeigt, dass Erbiumoxid in den Mengen als färbendes Metalloxid eingesetzt werden kann, ohne die Sintereigenschaften negativ zu beeinflussen. In einer weiteren bevorzugten Ausgestaltung der vorliegenden Erfindung ist das Gewichtsverhältnis von Terbiumoxid zu Cobaltoxid in wenigstens einer Pulverschicht, vorzugsweise in wenigstens 2 oder 3 Pulverschichten, insbesondere in jeder Pulverschicht im Bereich von 80:1 zu 5:1, vorzugsweise 75:1 bis 10:1 und speziell 60:1 bis 15:1. Insbesondere gute Farb- und Sintereigenschaften konnten mit den vorgenannten eingestellten Gewichtsverhältnissen erzielt werden speziell bei Einsatz von Tb₄O₇.

Jede Pulverschicht kann bevorzugt durch Vermischen von Basispulvern hergestellt werden. In einer bevorzugten Ausgestaltung erfolgt die Herstellung jeder Pulverschicht durch Vermischen von 2 oder mehr, vorzugsweise 3 oder mehr, insbesondere durch 4 oder mehr Basispulver. Das Verwenden einer begrenzten Menge von Basispulvern in unterschiedlichen Mengen für die Herstellung jeder Pulverschicht hat Vorteile hinsichtlich der Kompatibilität der einzelnen übereinander angeordneten Schichten und für deren Farbverlauf in der gesinterten dentalen Restauration. Geeignete Basispulver können beispielsweise von der Firma Tosoh bezogen werden.

Die gesinterten dentalen Restaurationen der vorliegenden Erfindung weisen bevorzugt einen graduellen Farbverlauf auf. In einer Ausgestaltung der Erfindung weist der Pressrohling farblich unterschiedliche Schichten auf und durch den Sintervorgang entsteht ein gradueller Farbverlauf.

In einer bevorzugten Ausführungsform der vorliegenden Erfindung weisen wenigstens 2, vorzugsweise alle Pulverschichten einen im Wesentlichen identischen Yttriumoxid Anteil auf.

Im Wesentlichen identischer Yttriumoxid-Anteil in 2 oder mehreren Schichten im Sinne der vorliegenden Erfindung bedeutet, dass die Differenz innerhalb der Schichten nicht mehr als 0,1 Mol-%, vorzugsweise nicht mehr als 0,05 Mol-%, insbesondere weniger als 0,01 Mol-% ausmacht.

In einer alternativen Ausgestaltung der vorliegenden Erfindung weist der gesinterte Formkörper einen Transluzenzverlauf auf. Vorzugsweise zeigt die erfindungsgemäße Restauration eine schichtweise Zunahme der Yttriumoxidmenge auf.

In einer bevorzugten Ausführungsform weisen die geschichteten Pressformkörper Pulverschichten auf mit jeweils mindestens 0,02 Gew.-% Al₂O₃.

Es hat sich als vorteilhaft herausgestellt, dass die Pulverschichten jeweils mindestens 0,02 Gew.-% Al₂O₃, vorzugsweise Al₂O₃ in einer Menge von 0,03 bis 0,15 Gew.-%, insbesondere Al₂O₃ in einer Menge von 0,03 bis 0,08 Gew.-% aufweisen. Die bevorzugte Menge Al₂O₃ in den einzelnen Pulverschichten führt zu einem verbesserten Stabilitäts- und Festigkeitsverhalten der erfindungsgemäßen Formkörper.

In einem weiteren Aspekt der vorliegenden Erfindung weisen wenigstens eine, vorzugsweise wenigstens 2 oder wenigstens 3 der Pulverschichten, insbesondere alle Pulverschichten Y₂O₃ und/oder Er₂O₃, vorzugsweise in einer Menge von wenigstens 3 Gew.-%, insbesondere von wenigstens 5 Gew.-% oder wenigstens 6 Gew.-% und insbesondere von 4,5 bis 12 Gew.-%, speziell von 6 bis 10 Gew.-% bezogen auf das Gesamtgewicht der Bestandteile der Pulverschicht, auf.

Das Y₂O₃ hat eine die Zirkoniumdioxidkristallphasen stabilisierende Funktion und ist kein färbendes Metalloxid im Sinne der vorliegenden Erfindung.

Das Erbiumoxid (Er₂O₃) hat eine stabilisierende Funktion für die ZrO2-Kristallphasen und ist ein färbendes Metalloxid im Sinne der vorliegenden Erfindung.

Bevorzugt weisen die Pulverschichten Zirkondioxid und/oder HfO₂ in einer Menge von wenigstens 89 Gew.-%, vorzugsweise in einer Menge von 89 bis 98 Gew.-%, insbesondere von 90 bis 96 Gew.-%, jeweils bezogen auf das Gesamtgewicht der Bestandteile der Pulverschicht, auf.

In einer bevorzugten Ausgestaltung umfassen die Pulverschichten Zirkoniumdioxid und Hafniumdioxid, besonders bevorzugt beträgt die Menge an Hafniumdioxid 0,1 bis 5 Gew.-%, speziell 0,5 bis 2,5 Gew.-%, bezogen auf das Gesamtgewicht von Zirkoniumdioxid und Hafniumdioxid.

In einer bevorzugten Ausgestaltung der vorliegenden Erfindung besteht der Pressformkörper aus 3 oder 4 oder insbesondere aus 5 verschiedenen keramischen Pulverschichten.

In einer weiteren bevorzugten Ausgestaltung der vorliegenden Erfindung besteht der Pressformkörper aus 5 keramischen Pulverschichten, wobei die erste Pulverschicht 20 bis 30 %, vorzugsweise 22 bis 28 %, die zweite Pulverschicht 10 bis 20 %, vorzugsweise 12 bis 18 %, die dritte Pulverschicht 15 bis 25 %, vorzugsweise 17 bis 23 %, die vierte Pulverschicht 10 bis 20 %, vorzugsweise 12 bis 18 % und die fünfte Pulverschicht 20 bis 30 %, vorzugsweise 22 bis 28 %, der Gesamtdicke der übereinander angeordneten Pulverschichten ausmacht und mit der Maßgabe, dass sich die Gesamtdicke auf 100 % ergänzt.

In einer weiteren bevorzugten Ausgestaltung der vorliegenden Erfindung besteht der Pressformkörper aus 5 keramischen Pulverschichten, wobei die erste Pulverschicht 5 bis 20 %, vorzugsweise 6 bis 18 %, die zweite Pulverschicht 5 bis 20 %, vorzugsweise 6 bis 18 %, die dritte Pulverschicht 10 bis 25 %, vorzugsweise 11 bis 20 %, die vierte Pulverschicht 10 bis 25 %, vorzugsweise 11 bis 20 % und die fünfte Pulverschicht 25 bis 65 %, vorzugsweise 30 bis 60 %, der Gesamtdicke der übereinander angeordneten Pulverschichten ausmacht und mit der Maßgabe, dass sich die Gesamtdicke auf 100 % ergänzt.

In einer Ausgestaltung der Erfindung wird der gesinterte Formkörper zunächst vorgesintert und durch subtraktive Verfahren bearbeitet und vorzugsweise anschließend in einem weiteren Schritt endgesintert wird.

In einer weiteren Ausgestaltung der vorliegenden Erfindung weist wenigstens eine Pulverschicht, vorzugsweise alle Pulverschichten zusätzlich organische Bestandteile, vorzugsweise in einer Menge von 3 - 6 Gew.-%, insbesondere in einer Menge von 4 - 5 Gew.-% auf. Als organische Bestandteile kommen insbesondere Bindemittel und Presshilfsmittel in Frage, die im Entbinderungsschritt thermisch leicht entfernt werden können. Geeignete Binder für Zirkoniumdioxid-Sinterpulver sind dem Fachmann bekannt. Hierzu zählt beispielsweise Polyvinylalkohol (PVA).

Bevorzugt weisen die Schichtpulver eine Schüttdichte unterhalb von 1,2 g/cm³ auf.

Es hat sich als vorteilhaft herausgestellt, Schichtpulver einzusetzen, die eine durchschnittliche Granulatgröße D₅₀ von 35 µm - 85 µm, bevorzugt von 40 µm - 80 µm und insbesondere von 50 µm - 70 µm oder 40 - 60 µm aufweisen. Gemessen werden die Granulatpulver trocken mittels Laserbeugung mittels eines Cilas Granulometer.

Üblicherweise weisen die anorganischen Bestandteile der Basispulver, also nach Entfernung der organischen Bestandteile, wie Bindemittel, etc., eine Partikelgröße D₅₀ von 0,1 bis 1 µm, bevorzugt von 0,2 µm - 0.8 µm und insbesondere von 0,2 µm - 0,7 µm, gemessen mittels Laserbeugung, auf. Es wurde gefunden, dass die Partikelgrößen einen positiven Beitrag zur Sinterung und insbesondere für die Farbübergänge zwischen den einzelnen Pulverschichten sorgen.

Der bevorzugt erfindungsgemäß zu sinternde Pressformkörper kann durch schichtweises übereinander Anordnen von 2 oder 3 oder insbesondere 4 oder fünf oder mehr keramischen Pulverschichten erhalten werden. Die Anordnung der Schichten kann beispielsweise in einem zylindrischen Behälter erfolgen unter Ausbildung von Scheiben oder Disks. Üblicherweise kann nach jedem Schichtauftrag ein uniaxiales Verpressen der Pulverschichten erfolgen. Dies kann beispielsweise durch einen Pressstempel erfolgen, wobei jedoch lediglich eine Vorverfestigung erfolgt. Das uniaxiale Verpressen der Schichten senkrecht zur Schichtoberfläche erfolgt bevorzugt bei einem Druck von 10-20 MPa, insbesondere 12-15 MPa.

In einer weiteren bevorzugten Ausgestaltung erfolgt das Verpressen der schichtweise übereinander angeordneten keramischen Pulverschichten zur Ausbildung eines Pressformkörpers durch Verpressen zunächst uniaxial und senkrecht zur Schichtoberfläche, vorzugsweise unter Ausbildung eines vorverdichteten Pressformkörpers mit einer Dichte unterhalb 2,8 g/cm³, vorzugsweise mit einer Dichte im Bereich von 2,5 - 2,7 g/cm³, bspw. 2,65 g/cm³. Das uniaxiale Vorverdichten kann zu einer besseren und innigeren Vermischung und damit gleichmäßigem Übergang zwischen den Schichten führen.

In einer weiteren bevorzugten Ausgestaltung erfolgt das Verpressen zur Herstellung des Pressformkörpers isostatisch, wobei das isostatische Verpressen vorzugsweise im Anschluss eines uniaxialen Vorverdichtens erfolgt, unter Ausbildung eines Pressformkörpers mit einer Dichte unterhalb von 3,4 g/cm³, insbesondere einer Dichte von 2,80 - 3,3 g/cm³, speziell mit einer Dichte von 2,85 - 3,25 g/cm³. Das isostatische Verpressen erfolgt bevorzugt, nachdem alle Schichten des Formpresskörpers angeordnet sind. Geeignete Drucke für das isostatische Verpressen liegen dabei gewöhnlich im Bereich von 500 - 10000 bar, bevorzugt im Bereich von 800 - 8000 bar, bspw. 1000 - 7000 bar oder 1000 - 3000 bar.

Die Dicken der einzelnen Pulverschichten des Pressformkörpers können variieren. In einer bevorzugten Ausgestaltung unterscheiden sich wenigstens zwei der keramischen Pulverschichten hinsichtlich ihrer Dicke. Vorzugsweise weisen wenigstens zwei der keramischen Pulverschichten des Pressformkörpers einen Dickenunterschied von wenigstens 5% auf. Typischerweise können die Pressformkörper in Form zylindrischer, kreisförmiger Scheiben mit Durchmessern im Bereich von 50 bis 200 mm, beispielsweise 75 bis 150 mm vorliegen. Die Gesamtdicke der zylindrischen Scheiben kann dabei beispielsweise im Bereich von 8 bis 40 mm, vorzugsweise von 10 bis 30 mm, speziell von 13 bis 25 mm liegen. Die Maße beziehen sich dabei auf den Pressformkörper im ungesinterten Zustand.

Hinsichtlich der Farbgestaltung und der anschließenden Bearbeitung hat es sich als vorteilhaft herausgestellt, dass wenigstens eine der äußeren keramischen Pulverschichten, vorzugsweise beide äußeren keramischen Pulverschichten des Pressformkörpers eine größere Dicke aufweist/aufweisen als eine zwischen den äußeren keramischen Pulverschichten liegende keramische Pulverschicht. Insbesondere bei der Verwendung der erfindungsgemäß hergestellten keramischen Formkörper für die Herstellung dentaler Restaurationen hat sich der zuvor beschriebene Schichtaufbau mit wenigstens einer dickeren äußeren Schicht als vorteilhaft erwiesen, da dies für die Bearbeitung in CAD/CAM Systemen oder anderen subtraktiven Bearbeitungsverfahren einen geeigneten Aufbau darstellt.

In einer speziell bevorzugten Ausgestaltung der vorliegenden Erfindung umfasst der Pressformkörper fünf keramische Pulverschichten, wobei die erste Pulverschicht 20 - 30%, vorzugsweise 22 - 28%, die zweite Pulverschicht 10 - 20%, vorzugsweise 12 - 18%, die dritte Pulverschicht 15 - 25%, vorzugsweise 17 - 23%, die vierte Pulverschicht 10 - 20%, vorzugsweise 12 - 18% und die fünfte Pulverschicht 20 - 30%, vorzugsweise 22 - 28% der Gesamtdicke der übereinander angeordneten Pulverschichten ausmacht und mit der Maßgabe, dass sich die Gesamtdicke auf 100% ergänzt.

In einer weiteren Ausgestaltung der vorliegenden Erfindung erfolgt die Vorsinterung bei einer Temperatur im Bereich von 950 - 1100°C, vorzugsweise von 980 - 1050°C unter Ausbildung eines vorgesinterten keramischen Formkörpers (Weißling). Üblicherweise erfolgt die Vorsinterung über einen Zeitraum, der ausreicht, um die vorhandenen Bindemittel zu entfernen und dem Pressformkörper eine genügende Festigkeit für die Bearbeitung durch subtraktive Verfahren zu verleihen. Die vorgesinterten und entbinderten Pressformkörper werden als Weißlinge bezeichnet.

In einer Ausgestaltung erfolgt die Vorsinterung zur Herstellung des Weißlings über einen Zeitraum oberhalb von 30 Minuten, bevorzugt oberhalb von 1 Stunde, insbesondere oberhalb von 20 Stunden oder oberhalb von 50 Stunden, beispielsweise 60 bis 200 Stunden oder 70 bis 150 Stunden.

Insbesondere zur Herstellung keramischer dentaler Restaurationen bietet es sich an, dass der vorgesinterte keramische Formkörper durch subtraktive Verfahren bearbeitet wird und vorzugsweise anschließend in einem weiteren Schritt endgesintert wird. Bei Anwendung subtraktiver Verfahren wird üblicherweise die Sinterschwindung mit einberechnet.

Die erfindungsgemäße Sinterung erfolgt üblicherweise bei Temperaturen oberhalb von 1350 °C, bevorzugt oberhalb von 1400 °C, speziell im Bereich von 1420 °C bis 1600 °C oder 1450 °C bis 1590°C oder 1480 bis 1580°C.

Die Sinterdauer für die erfindungsgemäße Sinterung erfolgt üblicherweise über einen Zeitraum von mehr als 4 Minuten, bevorzugt mehr als 5 Minuten, insbesondere im Bereich von 5 bis 120 Minuten.

In einer weiteren bevorzugten Ausgestaltung der vorliegenden Erfindung ist die erfindungsgemäße dentale Restauration erhältlich durch ein Verfahren umfassend die Schritte:
i) Bereitstellen von 2 oder 3 oder 4 oder 5 oder mehr, vorzugsweise farblich, unterschiedlichen keramischen Pulverschichten, die schichtweise übereinander angeordnet sind,
ii) Verpressen der schichtweise übereinander angeordneten keramischen Pulverschichten zur Ausbildung eines Pressformkörpers und
iii) Vorsintern des in Schritt ii) erhaltenen Formkörpers zur Ausbildung eines keramischen Weißlings,
iv) Subtraktive Bearbeitung des Weißlings zur Ausbildung einer dentalen Restauration,
   a) Sintern des keramischen Materials in einem Temperaturbereich oberhalb von 1350 °C und anschließendes
   b) Abkühlen der gesinterten dentalen Restauration, wobei die Abkühlung teilweise oder vollständig kontrolliert erfolgt.

In einer weiteren Ausgestaltung weisen die keramischen Pulverschichten jeweils eine voneinander verschiedene Zusammensetzung auf und wobei jede Pulverschicht, vorzugsweise
mindesten 85 Gew.-% ZrO₂ und/oder HfO2, Y₂O₃, Cobaltoxid, TiO₂ und
optional Er₂O₃ und/oder Terbiumoxid aufweist,
wobei die Gewichtsangaben jeweils bezogen auf die Pulverschicht sind.

In einer weiteren Ausgestaltung der Erfindung weist der Pressformkörper 2 oder mehr, vorzugsweise 3 oder mehr, besonders bevorzugt 4 oder mehr, speziell 5 keramischer Pulverschichten auf, wobei jede Pulverschicht
a) mindestens 85 Gew.-% ZrO₂ und/oder HfO₂,
b) 6 bis 10 Gew.-% Y₂O₃,
c) 0 bis 0,25 Gew.-% Terbiumoxid
d) 0,002 bis 1,0 Gew.-% TiO₂ und/oder
d1) 0,001 bis 0,2 Gew.-% Eisenoxid; und
e) optional 0 bis 0,7 Gew.-% Er₂O₃; und
f) optional Cobaltoxid aufweist,
wobei die Gewichtsangaben jeweils bezogen auf die Pulverschicht sind.

Das Basispulver umfasst bevorzugt Yttriumoxid in eine Menge von 5 bis 8 Gew.-%, vorzugsweise 6 bis 7 Gew.-%, Terbiumoxid in einer Menge von 0,1 bis 0,4 Gew.-%, vorzugsweise 0,1 bis 0,3 Gew.-% und Zirkoniumdioxid in einer Menge oberhalb von 80 Gew.-%, vorzugsweise von 82 Gew.-% bis 94 Gew.-%, speziell 84 Gew.-% bis 90 Gew.-%, jeweils bezogen auf das Gesamtgewicht des Basispulvers.

Ein weiterer Gegenstand der vorliegenden Erfindung ist ein Verfahren zur Herstellung einer dentalen Restauration umfassend die Schritte:
i) Bereitstellen von 2 oder 3 oder 4 oder 5 oder mehr, vorzugsweise farblich, unterschiedlichen keramischen Pulverschichten, die schichtweise übereinander angeordnet sind,
ii) Verpressen der schichtweise übereinander angeordneten keramischen Pulverschichten zur Ausbildung eines Pressformkörpers und
iii) Vorsintern des in Schritt ii) erhaltenen Formkörpers zur Ausbildung eines keramischen Weißlings,
iv) Subtraktive Bearbeitung des Weißlings zur Ausbildung einer dentalen Restauration,
   a) Sintern des keramischen Materials in einem Temperaturbereich oberhalb von 1350 °C und anschließendes
   b) Abkühlen der gesinterten dentalen Restauration, wobei die Abkühlung teilweise oder vollständig kontrolliert erfolgt.

Bevorzugte Ausgestaltung des erfindungsgemäßen Verfahrens wurden bereits obenstehend erläutert.

Die erfindungsgemäßen dentalen Restaurationen können in jeglicher Form vorliegen, beispielsweise als Kronen, Brücken, Onlays oder Inlays.

### Beispiele 1 bis 3 sowie Vergleichsbeispiel 1:

Tabelle 1 zeigt die Zusammensetzungen von 5 Schichten eines Pressformkörpers. Die Granulatgröße D₅₀ der Pulver liegt im Bereich von 40-80 µm. Die anorganischen Bestandteile der Basispulver weisen eine Partikelgröße D₅₀ von 0,2 bis 0,7 µm auf. Die Gewichtsangaben beziehen sich für jede Schicht auf das Gesamtgewicht der Pulverschicht.

Die Pressformkörper sind für die Verwendung in der Herstellung dentaler Restaurationen vorgesehen, so dass die Schichtzusammensetzungen entsprechend der Position im Zahn ausgestaltet sind. Die Zusammensetzung jeder Pulverschicht wird durch homogenes Vermischen in den angegebenen Mengen erzielt. Anschließend werden die Pulver schichtweise in eine zylinderförmige Pressform mit einem Durchmesser von 100 mm gegeben und eine Schichtdicke von 18 mm eingestellt. Die Pulverschichten werden uniaxial bei einem Druck von 13 MPa senkrecht zur Schichtoberfläche vorgepresst und anschließend isostatisch bei einem Druck von 2000 bar verpresst.

Nachfolgend erfolgt die Entbinderung und Vorsinterung bei ca. 1000 °C in einem Produktionsofen (Nabertherm N450) über einen Zeitraum von ca. 100 Stunden. Die so erhaltenen Weißlinge werden über CAD/CAM Systeme zu Zahnbrücke als dentale Restaurationen gefräst.

**Tabelle 1: Schichtzusammensetzungen:**

| **Schicht** | **Komponente** | **[Gew.-%]** |
|---|---|---|
| 1(inzisal) | Y₂O₃ | 7,99 |
| | TiO₂ | 0,12 |
| | Al₂O₃ | 0,028 |
| | Fe₂O₃ | 0,085 |
| | Co₃O₄ | 0,003 |
| | Er₂O₃ | 0,522 |
| | Organischer Binder | 4 |
| | ZrO₂ | ad 100 |
| | | |
| 2(inzisal) | Y₂O₃ | 7,45 |
| | TiO₂ | 0,30 |
| | Al₂O₃ | 0,035 |
| | Fe₂O₃ | 0,12 |
| | Co₃O₄ | 0,0025 |
| | Er₂O₃ | 0,626 |
| | Organischer Binder | 4 |
| | ZrO₂ | ad 100 |
| | | |
| 3(Dentin) | Y₂O₃ | 6,90 |
| | TiO₂ | 0,55 |
| | Al₂O₃ | 0,043 |
| | Fe₂O₃ | 0,15 |
| | Co₃O₄ | 0,0024 |
| | Er₂O₃ | 0,79 |
| | Organischer Binder | 4 |
| | ZrO₂ | ad 100 |
| | | |
| 4(Dentin) | Y₂O₃ | 6,39 |
| | TiO₂ | 0,75 |
| | Al₂O₃ | 0,050 |
| | Fe₂O₃ | 0,162 |
| | Co₃O₄ | 0,0032 |
| | Er₂O₃ | 0,93 |
| | Organischer Binder | 4 |
| | ZrO₂ | ad 100 |
| | | |
| 5(zervikal) | Y₂O₃ | 6,32 |
| | TiO₂ | 0,74 |
| | Al₂O₃ | 0,050 |
| | Fe₂O₃ | 0,160 |
| | Co₃O₄ | 0,004 |
| | Er₂O₃ | 1,044 |
| | Organischer Binder | 4 |
| | ZrO₂ | ad 100 |

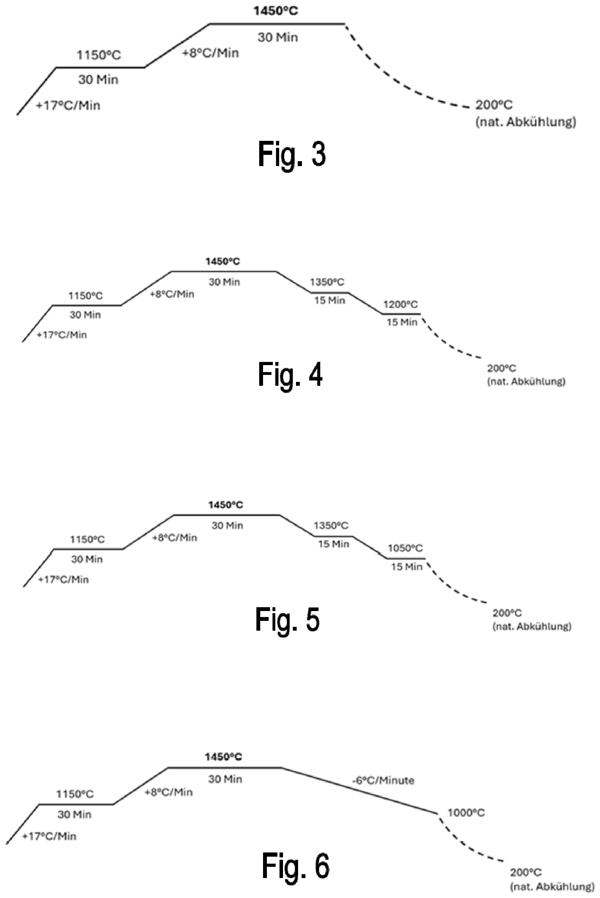

Diese vorgesinterten und bearbeiteten Weißlinge werden anschließend gemäß den in den Figuren 3 bis 6 dargestellten Temperaturverläufen in einem Dentalofen (VITA ZYRCOMAT 6100MS). gesintert und abgekühlt.

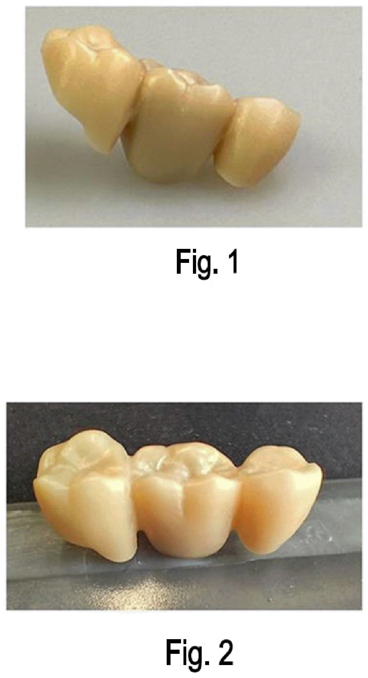

Fig. 3 zeigt die Sinterung des bearbeiteten Weißlings mit einer Sinterung bei 1450°C für 30 Minuten und anschließender unkontrollierter Abkühlung (Vergleichsbeispiel 1). Die erhaltene Zahnbrücke ist in Fig. 1 dargestellt. Es ist eine deutliche Graufärbung, insbesondere im Mittelbereich optisch erkennbar.

Fig. 4 zeigt die Sinterung des bearbeiteten Weißlings mit einer Sinterung bei 1450°C für 30 Minuten und anschließender kontrollierter Abkühlung (Beispiel 1). Bei der Abkühlung wurde eine Isotherme bei 1350°C und eine Isotherme bei 1200 °C für jeweils 15 Minuten eingehalten. Die erhaltene Zahnbrücke ist in Fig. 2 dargestellt. Es war optisch keine Vergrauung zu erkennen.

Fig. 5 zeigt die Sinterung des bearbeiteten Weißlings mit einer Sinterung bei 1450°C für 30 Minuten und anschließender kontrollierter Abkühlung (Beispiel 2).

Bei der Abkühlung wurde eine Isotherme bei 1350°C und eine Isotherme bei 1050 °C für jeweils 15 Minuten eingehalten. Die erhaltene Zahnbrücke zeigte optisch keine Vergrauung.

Fig. 6 zeigt die Sinterung des bearbeiteten Weißlings mit einer Sinterung bei 1450°C für 30 Minuten und anschließender kontrollierter Abkühlung (Beispiel 3). Bei der Abkühlung wurde eine graduelle Abkühlung mit einer Abkühlrate von 6 °C pro Minute von der Sintertemperatur bis 1000°C vorgenommen. Die erhaltene Zahnbrücke zeigte optisch keine Vergrauung.

**Tabelle 2: Messung der Cielab-Werte der dentalen Restaurationen mit Farbmessgerät (Zahnbrücke gemessen am Zwischenglied)**

| | L* | a* | b* | ΔE |
|---|---|---|---|---|
| Vergleichsbeispiel 1 | 60,8 | 3,7 | 40,5 | - |
| Beispiel 1 | 61,7 | 4,0 | 43,5 | 3,1 |
| Beispiel 2 | 63,0 | 4,0 | 44,4 | 4,5 |
| Beispiel 3 | 61,3 | 4,9 | 45,5 | 5,2 |

Das ΔE stellt die Differenz zwischen dem Vergleichsbeispiel und den erfindungsgemäßen Beispielen 1 bis 3 dar.

### Beispiele 4 bis 6 sowie Vergleichsbeispiel 2:

Tabelle 3 zeigt die Zusammensetzungen von 5 Schichten eines Pressformkörpers. Die Granulatgröße D₅₀ der Pulver liegt im Bereich von 40-80 µm. Die anorganischen Bestandteile der Basispulver weisen eine Partikelgröße D₅₀ von 0,2 bis 0,7 µm auf. Die Gewichtsangaben beziehen sich für jede Schicht auf das Gesamtgewicht der Pulverschicht.

Die Pressformkörper sind für die Verwendung in der Herstellung dentaler Restaurationen vorgesehen, so dass die Schichtzusammensetzungen entsprechend der Position im Zahn ausgestaltet sind. Die Zusammensetzung jeder Pulverschicht wird durch homogenes Vermischen in den angegebenen Mengen erzielt. Anschließend werden die Pulver schichtweise in eine zylinderförmige Pressform mit einem Durchmesser von 100 mm gegeben und eine Schichtdicke von 18 mm eingestellt. Die Pulverschichten werden uniaxial bei einem Druck von 13 MPa senkrecht zur Schichtoberfläche vorgepresst und anschließend isostatisch bei einem Druck von 2000 bar verpresst.

Nachfolgend erfolgt die Entbinderung und Vorsinterung bei ca. 1000 °C in einem Produktionsofen (Nabertherm N450) über einen Zeitraum von ca. 100 Stunden. Die so erhaltenen Weißlinge werden über CAD/CAM Systeme zu Kronen als dentale Restaurationen gefräst.

**Tabelle 3: Schichtzusammensetzungen:**

| **Schicht** | **Komponente** | **[Gew.-%]** |
|---|---|---|
| 1(inzisal) | Y₂O₃ | 7,93 |
| | TiO₂ | 0,11 |
| | Al₂O₃ | 0,028 |
| | Fe₂O₃ | 0,115 |
| | Co₃O₄ | 0,004 |
| | Er₂O₃ | 0,522 |
| | Organischer Binder | 4 |
| | ZrO₂ | ad 100 |
| | | |
| 2(inzisal) | Y₂O₃ | 7,38 |
| | TiO₂ | 0,32 |
| | Al₂O₃ | 0,035 |
| | Fe₂O₃ | 0,138 |
| | Co₃O₄ | 0,004 |
| | Er₂O₃ | 0,696 |
| | Organischer Binder | 4 |
| | ZrO₂ | ad 100 |
| | | |
| 3(Dentin) | Y₂O₃ | 6,84 |
| | TiO₂ | 0,54 |
| | Al₂O₃ | 0,043 |
| | Fe₂O₃ | 0,161 |
| | Co₃O₄ | 0,004 |
| | Er₂O₃ | 0,870 |
| | Organischer Binder | 4 |
| | ZrO₂ | ad 100 |
| | | |
| 4(Dentin) | Y₂O₃ | 6,39 |
| | TiO₂ | 0,74 |
| | Al₂O₃ | 0,050 |
| | Fe₂O₃ | 0,160 |
| | Co₃O₄ | 0,005 |
| | Er₂O₃ | 0,928 |
| | Organischer Binder | 4 |
| | ZrO₂ | ad 100 |
| | | |
| 5(zervikal) | Y₂O₃ | 6,42 |
| | TiO₂ | 0,72 |
| | Al₂O₃ | 0,050 |
| | Fe₂O₃ | 0,154 |
| | Co₃O₄ | 0,006 |
| | Er₂O₃ | 0,870 |
| | Organischer Binder | 4 |
| | ZrO₂ | ad 100 |

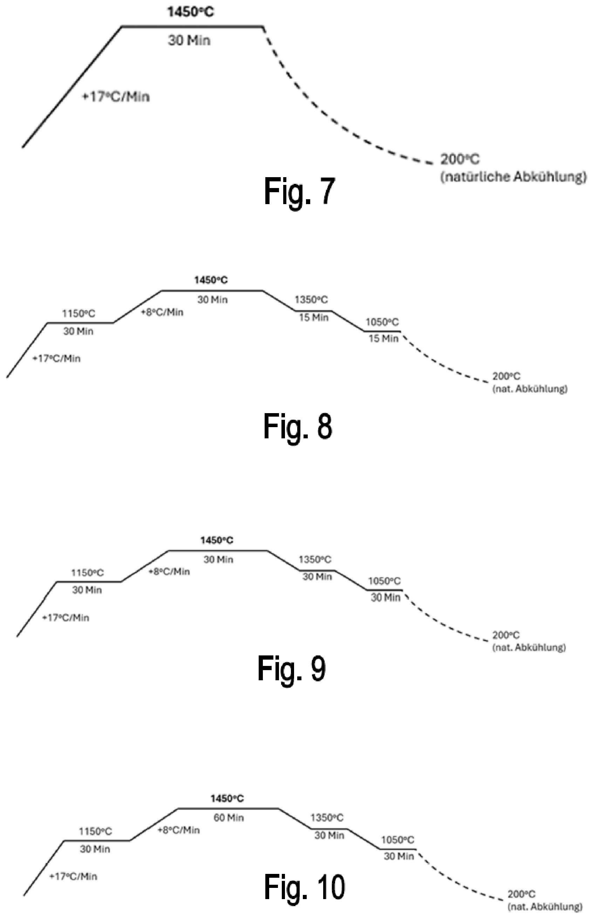

Diese vorgesinterten und bearbeiteten Weißlinge werden anschließend gemäß den in den Figuren 7 bis 10 dargestellten Temperaturverläufen in einem Dentalofen (VITA ZYRCOMAT 6100MS). gesintert und abgekühlt.

Fig. 7 zeigt die Sinterung des bearbeiteten Weißlings mit einer Sinterung bei 1450°C für 30 Minuten und anschließender unkontrollierter Abkühlung (Vergleichsbeispiel 2). Es ist eine deutliche Graufärbung der Krone erkennbar.

Fig. 8 zeigt die Sinterung des bearbeiteten Weißlings mit einer Sinterung bei 1450°C für 30 Minuten und anschließender kontrollierter Abkühlung (Beispiel 4). Bei der Abkühlung wurde eine Isotherme bei 1350°C und eine Isotherme bei 1050 °C für jeweils 15 Minuten eingehalten. Die erhaltene Krone ließ optisch keine Vergrauung zu erkennen.

Fig. 9 zeigt die Sinterung des bearbeiteten Weißlings mit einer Sinterung bei 1450°C für 30 Minuten und anschließender kontrollierter Abkühlung (Beispiel 4). Bei der Abkühlung wurde eine Isotherme bei 1350°C und eine Isotherme bei 1050 °C für jeweils 30 Minuten eingehalten. Die erhaltene Krone ließ optisch keine Vergrauung zu erkennen.

Fig. 10 zeigt die Sinterung des bearbeiteten Weißlings mit einer Sinterung bei 1450°C für 60 Minuten und anschließender kontrollierter Abkühlung (Beispiel 4). Bei der Abkühlung wurde eine Isotherme bei 1350°C und eine Isotherme bei 1050 °C für jeweils 30 Minuten eingehalten. Die erhaltene Krone ließ optisch keine Vergrauung zu erkennen.

**Tabelle 4: Messung der Cielab-Werte der dentalen Restaurationen mit Farbmessgerät (Krone gemessen an dickster Stelle)**

| | L* | a* | b* | ΔE |
|---|---|---|---|---|
| Vergleichsbeispiel 2 | 67,4 | 2,7 | 40,4 | - |
| Beispiel 4 | 69,5 | 3,3 | 43,5 | 3,8 |
| Beispiel 5 | 68,4 | 3,5 | 42,6 | 2,5 |
| Beispiel 6 | 68,1 | 3,6 | 43,6 | 3,4 |

Das ΔE stellt die Differenz zwischen dem Vergleichsbeispiel und den erfindungsgemäßen Beispielen 4 bis 6 dar.

### Beispiele 7 sowie Vergleichsbeispiel 3:

Tabelle 5 zeigt die Zusammensetzungen von 5 Schichten eines Pressformkörpers. Die Granulatgröße D₅₀ der Pulver liegt im Bereich von 40-80 µm. Die anorganischen Bestandteile der Basispulver weisen eine Partikelgröße D₅₀ von 0,2 bis 0,7 µm auf. Die Gewichtsangaben beziehen sich für jede Schicht auf das Gesamtgewicht der Pulverschicht.

Die Pressformkörper sind für die Verwendung in der Herstellung dentaler Restaurationen vorgesehen, so dass die Schichtzusammensetzungen entsprechend der Position im Zahn ausgestaltet sind. Die Zusammensetzung jeder Pulverschicht wird durch homogenes Vermischen in den angegebenen Mengen erzielt. Anschließend werden die Pulver schichtweise in eine zylinderförmige Pressform mit einem Durchmesser von 100 mm gegeben und eine Schichtdicke von 18 mm eingestellt. Die Pulverschichten werden uniaxial bei einem Druck von 13 MPa senkrecht zur Schichtoberfläche vorgepresst und anschließend isostatisch bei einem Druck von 2000 bar verpresst.

Nachfolgend erfolgt die Entbinderung und Vorsinterung bei ca. 1000 °C in einem Produktionsofen (Nabertherm N450) über einen Zeitraum von ca. 100 Stunden. Die so erhaltenen Weißlinge werden über CAD/CAM Systeme zu dentalen Brücken als dentale Restaurationen gefräst.

**Tabelle 5: Schichtzusammensetzungen:**

| **Schicht** | **Komponente** | **[Gew.-%]** |
|---|---|---|
| 1(inzisal) | Y₂O₃ | 6,53 |
| | Al₂O₃ | 0,05 |
| | Fe₂O₃ | 0,101 |
| | Co₃O₄ | 0,010 |
| | Er₂O₃ | 0,092 |
| | Organischer Binder | 4 |
| | ZrO₂ | ad 100 |
| | | |
| 2 | Y₂O₃ | 6,49 |
| | Al₂O₃ | 0,05 |
| | Fe₂O₃ | 0,117 |
| | Co₃O₄ | 0,010 |
| | Er₂O₃ | 0,138 |
| | Organischer Binder | 4 |
| | ZrO₂ | ad 100 |
| | | |
| 3 | Y₂O₃ | 6,50 |
| | Al₂O₃ | 0,05 |
| | Fe₂O₃ | 0,130 |
| | Co₃O₄ | 0,009 |
| | Er₂O₃ | 0,184 |
| | Organischer Binder | 4 |
| | ZrO₂ | ad 100 |
| | | |
| 4 | Y₂O₃ | 6,41 |
| | Al₂O₃ | 0,05 |
| | Fe₂O₃ | 0,145 |
| | Co₃O₄ | 0,010 |
| | Er₂O₃ | 0,230 |
| | Organischer Binder | 4 |
| | ZrO₂ | ad 100 |
| | | |
| 5 (zervikal) | Y₂O₃ | 6,33 |
| | Al₂O₃ | 0,05 |
| | Fe₂O₃ | 0,149 |
| | Co₃O₄ | 0,012 |
| | Er₂O₃ | 0,276 |
| | Organischer Binder | 4 |
| | ZrO₂ | ad 100 |

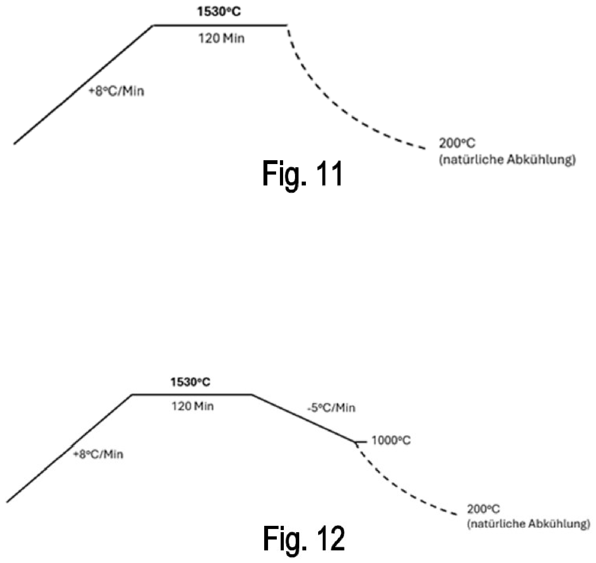

Diese vorgesinterten und bearbeiteten Weißlinge werden anschließend gemäß den in den Figuren 11 12 dargestellten Temperaturverläufen in einem Dentalofen (VITA ZYRCOMAT 6100MS). gesintert und abgekühlt.

Fig. 11 zeigt die Sinterung des bearbeiteten Weißlings mit einer Sinterung bei 1530°C für 120 Minuten und anschließender unkontrollierter Abkühlung (Vergleichsbeispiel 3). Es ist eine verminderte Farbsättigung der Brücke erkennbar.

Fig. 12 zeigt die Sinterung des bearbeiteten Weißlings mit einer Sinterung bei 1530°C für 120 Minuten und anschließender kontrollierter Abkühlung (Beispiel 7). Bei der Abkühlung wurde eine graduelle Abkühlung mit einer Abkühlrate von 5°C von 1530 °C bis 1000°C durchgeführt. Die erhaltene Brück zeigte optisch eine verbesserte Farbsättigung.

**Tabelle 6: Messung der Cielab-Werte der dentalen Restaurationen mit Farbmessgerät (Zahnbrücke gemessen am Zwischenglied)**

| | L* | a* | b* | ΔE |
|---|---|---|---|---|
| Vergleichsbeispiel 3 | 73,3 | 5,2 | 37,3 | - |
| Beispiel 7 | 68,5 | 5,8 | 41,1 | 6,2 |

Das ΔE stellt die Differenz zwischen dem Vergleichsbeispiel und dem erfindungsgemäßen Beispiel 7 dar. Es konnte somit eine verbesserte Farbsättigung für das erfindungsgemäße Beispiel 7 gemessen werden.

## Patentansprüche

1. Dentale Restauration erhältlich durch ein Verfahren umfassend die Schritte:
a) Sintern eines keramischen Materials enthaltend Titandioxid (TiO₂) und/oder Eisenoxid und anschließendes
b) Abkühlen der gesinterten dentalen Restauration, vorzugsweise auf Raumtemperatur, wobei die Abkühlung teilweise oder vollständig kontrolliert erfolgt, wobei das kontrollierte Abkühlen einen Temperaturabschnitt umfasst, der mit einer Abkühlrate von 1 bis 25 °C/min erfolgt; oder
das kontrollierte Abkühlen stufenweise erfolgt und 2 oder mehr Isothermen mit einer Haltedauer von vorzugsweise mindestens 5 Minuten umfasst.

2. Dentale Restauration gemäß Anspruch 1, **dadurch gekennzeichnet, dass** das Sintern in einem Temperaturbereich oberhalb von 1350 °C, vorzugsweise von 1400 °C bis 1700 °C, insbesondere von 1420°C bis 1650°C erfolgt.

3. Dentale Restauration gemäß einem oder mehreren der vorangehenden Ansprüche, **dadurch gekennzeichnet, dass** das kontrollierte Abkühlen in einem oberen Temperaturabschnitt, vorzugsweise von der Sintertemperatur bis zu etwa 800°C, besonders bevorzugt von Sintertemperatur bis etwa 1000°C, erfolgt.

4. Dentale Restauration gemäß einem oder mehreren der vorangehenden Ansprüche, **dadurch gekennzeichnet, dass** das kontrollierte Abkühlen einen Temperaturabschnitt umfasst, der mit einer Abkühlrate von 2 bis 15°C/min, insbesondere von 3 bis 10°C/min oder 3 bis 7 °C/min erfolgt.

5. Dentale Restauration gemäß einem oder mehreren der vorangehenden Ansprüche, **dadurch gekennzeichnet, dass** das kontrollierte Abkühlen stufenweise erfolgt, wobei wenigstens eine Isotherme, vorzugsweise im Temperaturbereich oberhalb von 800°C, speziell oberhalb von 1000°C, insbesondere über einen Zeitraum von mehr als 5 Minuten, insbesondere von mehr als 10 Minuten oder in einem Bereich von 10 bis 45 Minuten eingehalten wird.

6. Dentale Restauration gemäß einem oder mehreren der vorangehenden Ansprüche, **dadurch gekennzeichnet, dass** das kontrollierte Abkühlen stufenweise erfolgt und 2 oder mehr Isothermen mit einer Haltedauer von mindestens 10 Minuten und speziell zwischen 10 Minuten und 120 Minuten, beispielsweise 15 bis 45 Minuten, umfasst.

7. Dentale Restauration gemäß einem oder mehreren der vorangehenden Ansprüche, **dadurch gekennzeichnet, dass** das kontrollierte Abkühlen ganz oder teilweise in einem Temperaturbereich von 1400 bis 800°C erfolgt.

8. Dentale Restauration gemäß einem oder mehreren der vorangehenden Ansprüche, **dadurch gekennzeichnet, dass** das keramische Material mindestens 85 Gew.-% ZrO₂ und/oder HfO₂ umfasst, wobei die Gewichtsangabe bezogen auf das Gesamtgewicht des keramischen Materials ist.

9. Dentale Restauration gemäß einem oder mehreren der vorangehenden Ansprüche, **dadurch gekennzeichnet, dass** das keramische Material Eisenoxid in einer Menge von 0,001 bis 0,2 Gew.-%, vorzugsweise von 0,01 bis 0,18 Gew.-%, aufweist.

10. Dentale Restauration gemäß einem oder mehreren der vorangehenden Ansprüche, **dadurch gekennzeichnet, dass** die Restauration einen Farbverlauf und/oder Transluzenzverlauf aufweist.

11. Dentale Restauration gemäß einem oder mehreren der vorangehenden Ansprüche, **dadurch gekennzeichnet, dass** das keramische Material Er₂O₃, vorzugsweise in einer Menge von 0 bis weniger 1 Gew.-%, insbesondere von 0,1 bis weniger als 0,9 Gew.-% oder von 0,2 Gew.-% bis 0,8, insbesondere von 0,25 bis 0,7 Gew.-%, speziell von 0,3 bis 0,6 Gew.-%, jeweils bezogen auf das Gesamtgewicht des keramischen Materials, und/oder
das keramische Material Terbiumoxid in einer Menge von 0,001 bis 0,25 Gew.-%, vorzugsweise von 0,002 bis 0,19 Gew.-%, insbesondere von 0,003 bis 0,17 Gew.-%, speziell 0,01 bis 0,15 Gew.-% aufweist, wobei die Gewichtsangaben (Gew.-%) jeweils bezogen sind auf das Gesamtgewicht des keramischen Materials.

12. Dentale Restauration gemäß einem oder mehreren der vorangehenden Ansprüche, **dadurch gekennzeichnet, dass** das keramische Material Titandioxid in einer Menge von 0,001 bis 1,0 Gew.-%, vorzugsweise von 0.002 bis 0.8 Gew.-%, oder 0,002 bis 0,6 Gew.-%, insbesondere von 0,005 bis 0,5 Gew.-%, aufweist, wobei die Gewichtsangaben (Gew.-%) jeweils bezogen sind auf das Gesamtgewicht des keramischen Materials.

13. Dentale Restauration gemäß einem oder mehreren der vorangehenden Ansprüche erhältlich durch ein Verfahren umfassend die Schritte:
i) Bereitstellen von 2 oder 3 oder 4 oder 5 oder mehr, vorzugsweise farblich, unterschiedlichen keramischen Pulverschichten, die schichtweise übereinander angeordnet sind,
ii) Verpressen der schichtweise übereinander angeordneten keramischen Pulverschichten zur Ausbildung eines Pressformkörpers und
iii) Vorsintern des in Schritt ii) erhaltenen Pressformkörpers zur Ausbildung eines keramischen Weißlings,
iv) Subtraktive Bearbeitung des Weißlings zur Ausbildung einer dentalen Restauration,
a) Sintern des keramischen Materials in einem Temperaturbereich oberhalb von 1350 °C und anschließendes
b) Abkühlen der gesinterten dentalen Restauration, wobei die Abkühlung teilweise oder vollständig kontrolliert erfolgt.

14. Dentale Restauration gemäß Anspruch 13, **dadurch gekennzeichnet, dass**
die keramischen Pulverschichten jeweils eine voneinander verschiedene Zusammensetzung aufweisen und wobei jede Pulverschicht, vorzugsweise
mindesten 85 Gew.-% ZrO₂ und/oder HfO2, Y₂O₃, Terbiumoxid, TiO₂ und
optional Er₂O₃ aufweist,
wobei die Gewichtsangaben jeweils bezogen auf die Pulverschicht sind.

15. Dentale Restauration gemäß einem oder mehreren der vorangehenden Ansprüche 13 oder 14, **dadurch gekennzeichnet, dass** der Pressformkörper 2 oder mehr, vorzugsweise 3 oder mehr, besonders bevorzugt 4 oder mehr, speziell 5 keramischer Pulverschichten aufweist, wobei jede Pulverschicht
a) mindestens 85 Gew.-% ZrO₂ und/oder HfO₂,
b) 6 bis 10 Gew.-% Y₂O₃,
c) 0 bis 0,25 Gew.-% Terbiumoxid,
d) 0,002 bis 1,0 Gew.-% TiO₂ und/oder
d1) 0,001 bis 0,2 Gew.-% Eisenoxid; und
e) optional 0 bis 0,7 Gew.-% Er₂O₃; und
f) optional Cobaltoxid aufweist,
wobei die Gewichtsangaben jeweils bezogen auf die Pulverschicht sind.
